# EUROPEAN PATENT SPECIFICATION

(11) **EP 2 266 655 B1**
(45) Date of publication and mention of the grant of the patent: **07.08.2013**
(21) Application number: 10165167.7
(22) Date of filing: 08.06.2010
(51) Int. Cl.: A61M 25/06

(54) **Variable diameter tubular structure for a biomedical use**
Introducerkörper mit variablem Durchmesser zur biomedizinischen Verwendung
Structure tubulaire de diamètre variable pour utilisation biomédicale

(30) Priority: 23.06.2009 IT MI20091102
(43) Date of publication of application: 29.12.2010
(73) Proprietor: N.G.C. Medical S.p.A., 22060 Novedrate (CO) (IT)
(72) Inventor: Pini, Patrizia, I-22060, Novedrate (CO) (IT); Cremascoli, Paolo, I-22060, Novedrate (CO) (IT); Silva, Pedro, I-22060, Novedrate (CO) (IT)
(74) Representative: Cicogna, Franco

(56) References cited:
- WO-A1-2004/037333
- JP-A- 2006 239 236
- US-A- 4 921 479
- US-A1- 2002 032 459

## Description

### BACKGROUND OF THE INVENTION

The present invention relates to variable diameter tubular structure for a biomedical use.

As is known, in the biomedical field artificial ducts for conveying biologic liquids, drugs and other medical devices therethrough are broadly used and include, for example, either implantable or temporary ducts, to be used in hospital diagnostic or surgical operations, in which it is necessary to convey a medical device from an operating environment to a human body.

This operation is performed by a so-called introducer device, the function of which is that of providing and holding in a patent condition a communication channel communicating with the patient body, and usually held in the patient body through the overall duration of the surgical operation or for a larger period.

The most common access ways are those to the haematic ducts (for example for performing a vascular catheterization), the myocardium (for example for transapically locating a cardiac valve) and, in general to other biologic ducts (for example for locating biliary, urethral and ureteral stents).

Said introducer device comprises plastics material tubes either including or not a sealing valve, and thereinto are introduced surgical devices and having an outer diameter either less than or equal to the inner diameter of the introducer device.

Said introducer devices should provide a low inner friction to facilitate the conveying therethrough of said medical devices from atraumatic tip and body portions, to prevent patient tissues from being damaged, and should moreover have such a radial stiffness as not to be bent under operating pressures, and further optionally including a sealing valve and related side arm assembly for performing guiding withdrawing and/ monitoring operations, in which said introducer device is introduced into the patient body by a corresponding dilatator assembly, that is a tube having a tapering tip designed for easily entering the patient tissues to provide the introducer and dilatator device with a comparatively high radial strength.

At the end of the introducing operation, the dilatator assembly is withdrawn from the patient body, while leaving therein only the introducer.

In some surgical operations it is necessary to change an introducer device and replace it by another having either a larger or smaller diameter.

Up to now, the above operation would involve removing the introducer and locating a new one.

See e.g. US 2002/0032459 or WO 2004/037333

### SUMMARY OF THE INVENTION

Accordingly, the aim of the present invention is to provide such a tubular structure, as claimed in claim 1, having a variable diameter, that is a starting rest diameter which may be easily enlarged, without applying any radial force, to recover said rest diameter as the structure is released or is not subjected to an outer force.

Within the scope of the above mentioned aim, a main object of the invention is to provide such a variable diameter tubular structure which may be used as an introducer body and for performing other biomedical operations, for example to provide a permanent implant, a functional part of an artificial ventricle or a sphincter assembly.

Another object of the invention is to provide such a variable diameter tubular structure suitable to greatly improve related surgical or implant procedures and greatly reduce the number of introducer devices to be used in said procedure, with a consequent reduction of the operation, bleeding time and vessel or tissue traumatic stress.

Another object of the invention is to provide such a variable diameter tubular structure allowing to greatly reduce the number of operations to implant different diameter artificial channel elements.

Another object of the present invention is to provide such a variable diameter tubular structure facilitating a closure of a puncture place.

Another object of the present invention is to provide such a variable diameter tubular structure allowing to greatly reduce vessel wall traumas.

Yet another object of the present invention is to provide such a variable diameter tubular structure which is very reliable and safe in operation.

According to one aspect of the present invention, the above mentioned aim and objects, as well as yet other objects, which will become more apparent hereinafter, are achieved by a biomedical variable diameter tubular structure, as claimed in claim 1, **characterized in that** said structure comprises an extruded tube, which is longitudinally cut and then thermoformed to define a longitudinal region having overlapping walls sliding with respect to one another in a radial direction, said walls moving away as an article having a size larger than a rest diameter of said tube passes through said tube.

According to a further aspect of the invention, said tube is coupled to a resilient sheath extending through the overall length of said tube.

Up to now in the medical field were not used polymeric materials having a shape memory feature and a good structural stiffness, which characteristics, on the other hand, were well known in steel and nickel-titanium alloy mesh patterns.

The present invention provides a plastics material tube having a variable diameter which may be advantageously used in the medical field.

### BRIEF DESCRIPTION OF THE DRAWINGS

Further characteristics and advantages of the present invention will become more apparent hereinafter from the following disclosure of a preferred, though not exclusive, embodiment of the invention, which is illustrated, by way of an indicative, but not limitative, example in the accompanying drawings, where:
Figure 1 shows a cross sectional view of a variable diameter tube structure according to an example respectively in a rest and in an expanded condition thereof;
Figure 2 shows, in cross-section, another exemplary variable diameter structure in a rest and expanded condition thereof;
Figure 3 is a cross-sectional view of an embodiment of the thermoformed tube structure according to the present invention, including inward directed rough portions;
Figure 4 is a further cross-sectional view of another embodiment of the thermoformed tube structure according to the present invention including outwardly directed rough portions;
Figure 5 is a perspective view showing the guide, dilatator and introducer assembly ;
Figure 6 is a further perspective view showing the introducer device in a rest condition thereof, without a dilatator engaged therein;
Figures 7 and 7a are further perspective views showing the introducer device with an enlarged diameter, being enlarged by causing a larger diameter catheter to slide therein;
Figure 8 is a cross sectional view of the tube including an outer tube sheath in a rest condition thereof;
Figure 9 is a further cross-sectional view of the tube in an enlarged condition thereof, the inner device being not shown;
Figure 10 is yet another perspective view showing a deformation of the introducer with the device engaged therein and having an enlarged diameter only at a portion of its overall length; and
Figure 11 shows an introducer device having a tip protected by a dilatator and a diameter equal to or larger than that of the dilatator in a rest condition thereof.

### DESCRIPTION OF THE PREFERRED EMBODIMENTS

With reference to the number references of the above mentioned figures, the variable diameter tubular structure according to the present invention, which has been generally indicated by the reference number 1, is made starting from an extruded tube which is properly modified and thermoformed and finally coupled to a resilient sheath assembly 2 extending through the overall length of the device body.

The expansion of the tube is achieved by providing a region 3 having radially sliding walls, which are moved away as an article having a size larger than the rest diameter of the tube is caused to pass through the latter.

For making the tube to a required or set contour, the following operations are performed:
- an extrusion of the tube with a set thickness and diameter, for example a thickness of 0.5 and a diameter of 6 mm;
- a longitudinal cutting of the tube;
- an overlapping of the two tube open portions to a tube rest diameter, for example 5 mm, with a proper thermoforming of thus achieved contour.

Thus, by the above operations, a tube is achieved having an inner rest diameter corresponding to a minimum target diameter and which may be easily enlarged.

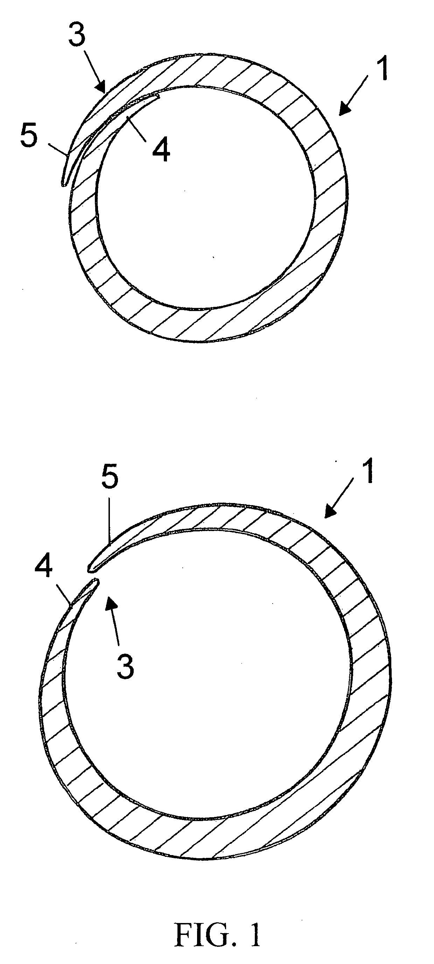

A preferred configuration of the extruded and thermoformed tube is shown in figure 1, in which the tube has a variable thickness and the two open flaps or edges thereof, indicated by the reference numbers 4 and 5, are smugly overlapped thereby providing a precise thickness of said tube in its minimum diameter configuration.

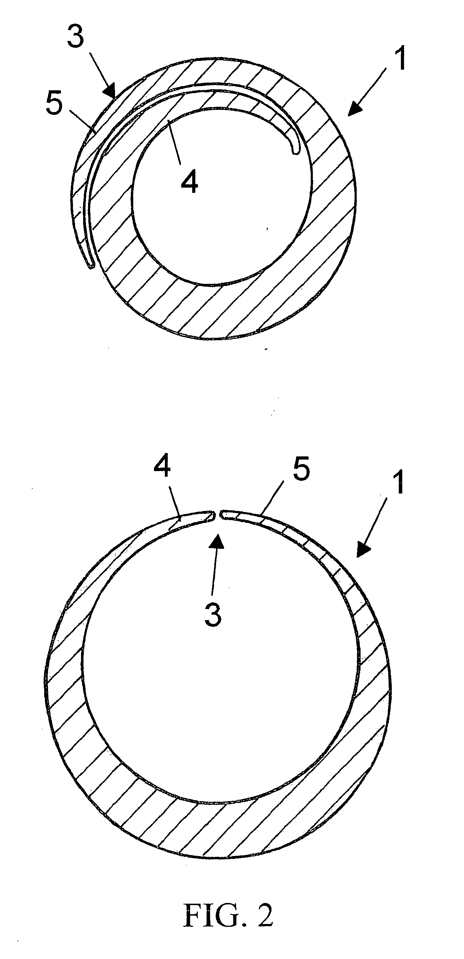

Another similar solution, but with the tube having an eccentric thickness, is shown in figure 2.

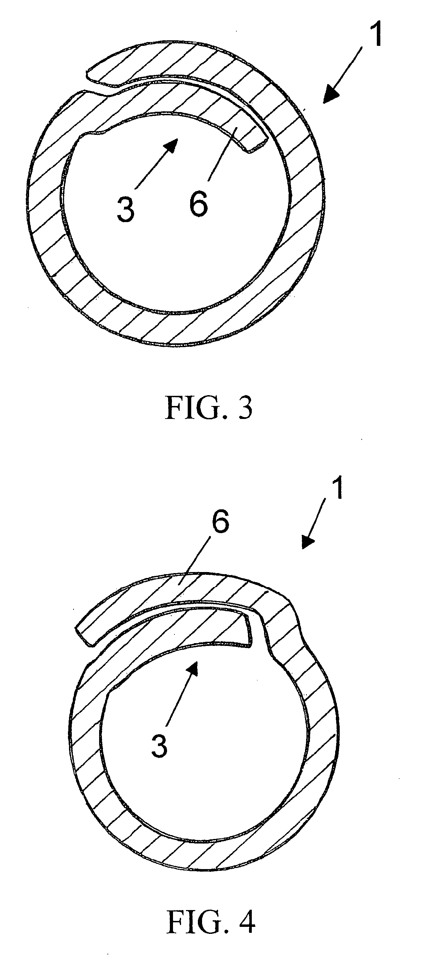

The inventive tube contour is of the type shown in figures 3 and 4, wherein the tube is extruded to or with an even thickness, the movable flap or tube portions being overlapped, and the assembly being then thermoformed to provide a non linear contour or profile, including rough portions which may be directed either inward and/or outward depending on the thermoforming process.

Because the tube is extruded to a small thickness, for example 0.1 mm, said rough portions will be very small and generally acceptable.

The material forming the variable diameter tubular structure according to the present invention is preferably polyamide, PTFE or PE.

To insulate the tube inside with respect to the encompassing environment, for example with respect to arterial blood, a resilient membrane 2 is applied to the tube contour.

Advantageously, for facilitating the operation, said membrane 2 is extended through the overall length and profile of said tube, said membrane 2 being made of a polyurethane material and being applied to the tube in a tube rest diameter condition by a dipping coating method.

If desired, said membrane 2 may also be made by extruding and glueing operating steps.

A primer coating and other surface treatments may also be used.

The thus made tube, accordingly, will comprise two tube layers: a stiffener layer, providing the tube duct or lumen, and a more resilient one, preventing the sliding tube portions from detaching and communications between the tube inner lumen and outer environment from occurring.

The tube, on the other hand, may be easily enlarged by engaging a device having a diameter larger than that the tube rest diameter therein.

A withdrawing of the inner device allowing the resilient membrane 2 to "recover" to their starting conditions the tube movable portions, thereby the tube will return to its rest diameter.

The finished tube lubricating property may be improved by suitable lubricating or lining materials, of a type conventionally used in the medical field, thereby the tube may be easily used as a tube body for an introducer device.

In such an application, a proximal part of said tube is sealed to a valve 8 holding said tube in its desirable maximum diameter condition.

If the tube is used to make an introducer, then it is necessary to provide a tube tip 9 tapering to the dilatator, thereby preventing it from rubbing against the tissues traversed thereby.

This result may be achieved by properly thermoforming the tip region of the tube.

Moreover it is preferred to provide a proper adhesion between the two tube layers, the tip being in this case made of the stiffener layer only.

The inventive tube has advantageously a very small wall thickness, and may be enlarged in a "modulable" and adjustable manner, that is in a non permanent manner through the overall length thereof, while having a very low cost.

The variable tubular structure according to the present invention may be further advantageously used to make a vascular introducer device body, as indicated by 100 in the drawing figures.

In this case, the tube will have a tube end fitted to a holding valve 8 having a side arm 10 and an atraumatic blunted tip 9, the valve being a haemostatic valve comprising a stiff fitting including a silicone diaphragm insulating the outside and inside of the vessel, but allowing medical devices to pass therethrough, said silicone diaphragm being precut, for example in the form a central star, the cuts snugly adhering to the device passing therethrough.

In such a construction, the valve will be properly calibrated for allowing devices having a maximum target diameter, for example of 7 mm, to easily pass therethrough.

The tube fitted to the valve, accordingly, will be opened to a target maximum diameter of 7 mm, whereas the remaining portion of the introducer body will have the target minimum diameter for example of 5 mm.

The introducer, accordingly, will have, at said valve, a diameter transition conical region 11.

The vascular introducer 100 is engaged in the vessel through the patient tissue and vessel wall, by a well known Seldinger's procedure, in which the vessel is punctured, the guide engaged therein and then the introducer device preassembled on a dilatator being further engaged.

The introducer device may be used in a like manner, by using a dilatator corresponding to the minimum target diameter, for example of 5 mm.

Such a dilatator is a commercially available one, as well as the other conventionally used devices.

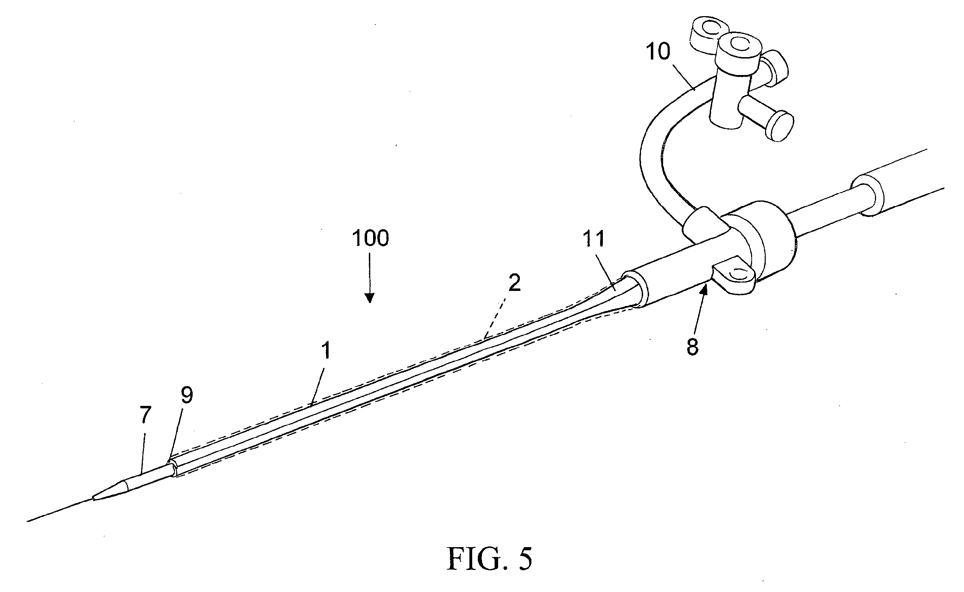

Figure 5 shows the guide + dilatator + introducer in an assembled condition thereof.

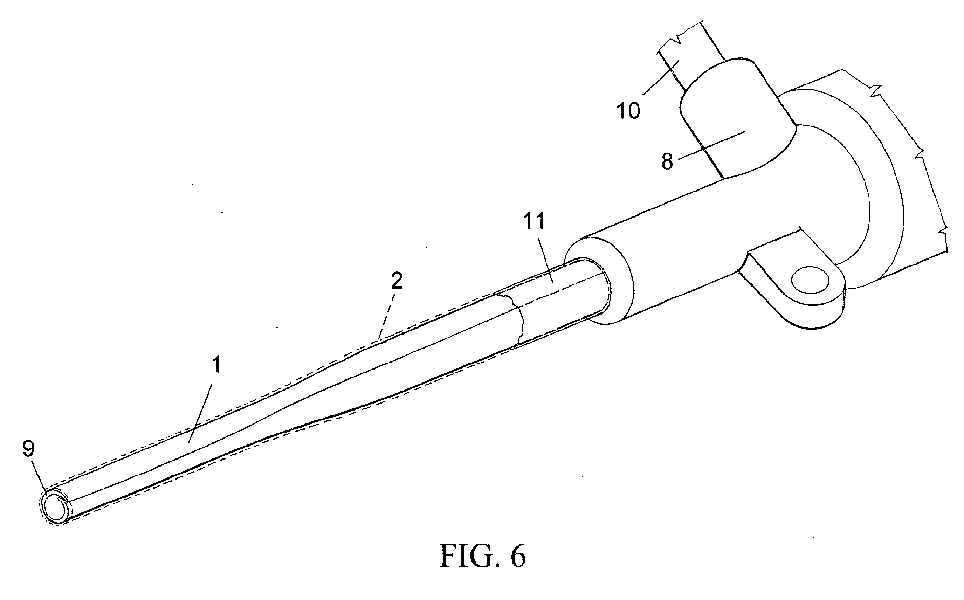

Figure 6 shows the introducer device in a rest condition thereof, without any dilatator engaged therein.

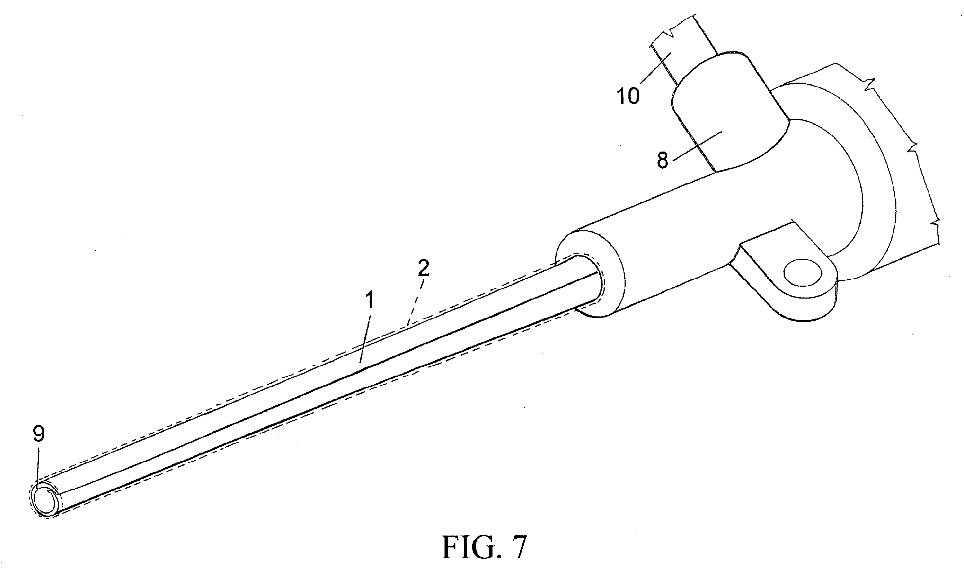

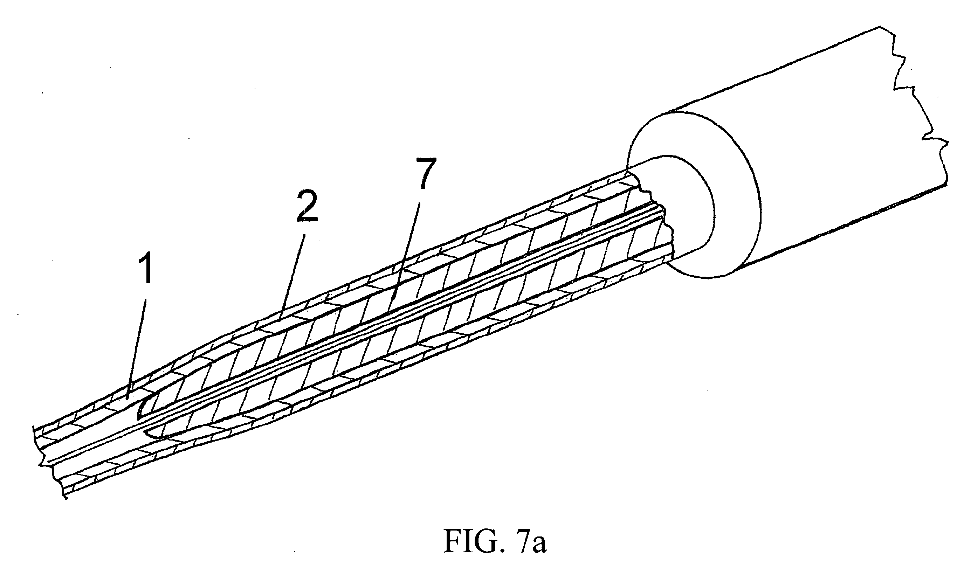

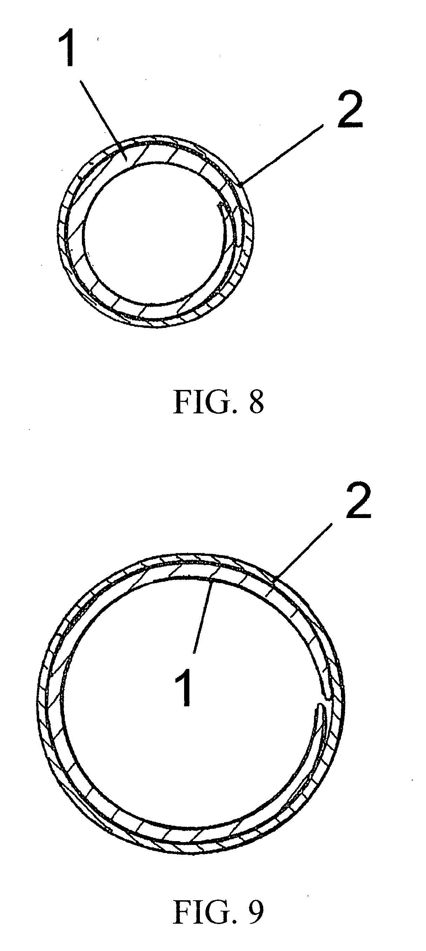

The introducer being then enlarged to the larger diameter, for example a maximum diameter of 7 mm, by causing a catheter to slide therethrough, said catheter having a larger diameter, as is shown in figures 7-9.

A further advantage of the structure according to the present invention is that it does not require the use of a second dilatator.

Yet another advantage is that the enlargement of the introducer and, accordingly, of the tissues therethrough said introducer passes, is limited in the time and performed only as it is necessary.

Actually, the introduced device may have a larger diameter only at a portion of its overall length.

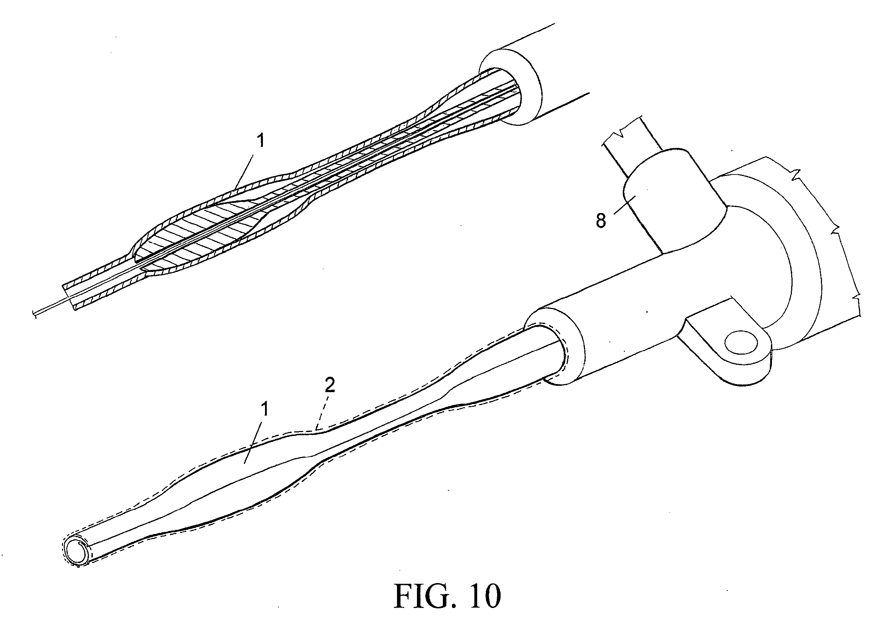

In this case the introducer is deformed as shown in figure 10.

If it is desired to achieve a channel having a larger permanent diameter, that is a diameter larger than the rest diameter, it is preferred to restrain the tube by engaging therein a second tube.

Since the resilient membrane may cause problems related to the introducer tip tapering and, accordingly, make the introducer making method expensive, it is possible to provide a substantially truncated introducer tip and use a dilatator having a modified tip adapted to "protect the tissue against the introducer tip portion to be withdrawn owing to the introducer tube elastic feature.

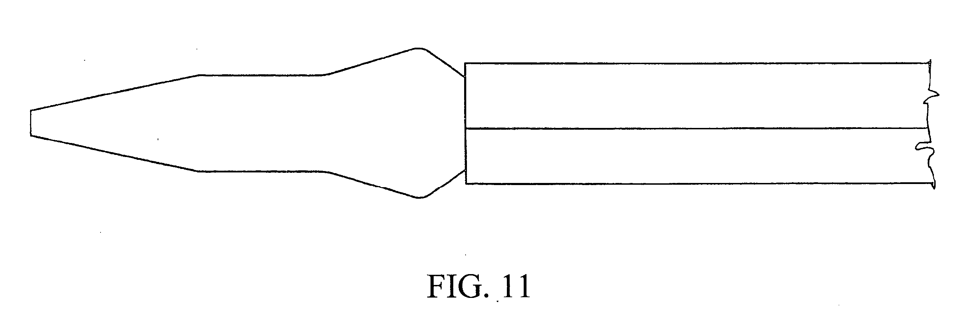

In this case the dilatator will have a maximum diameter either larger than or equal to the introducer rest diameter, and less than the enlarged introducer diameter, as shown in figure 11.

The variable diameter tubular structure according to the present invention is susceptible to several modifications and variations, all coming within the inventive idea scope.

If desired, an exemplary tube may also not be lined by a resilient membrane, for example for a use in a dry environment, to prevent any material infiltration problems from occurring.

Moreover, the resilient membrane, instead of being arranged outside the tube, may be also arranged therewithin, and may also be made by an independent making process and easily sealed/glued to the tube.

The membrane, in particular, may also be made by an extruding process, as a first or a second layer of the rigid tube.

The membrane may also be made by subjecting the tube to a dipping method in its as extruded or it is both extruded and thermoformed condition.

The tube may also be coated by hydrophilic coating material on the inner and/or outer walls thereof, to improve the sliding capability of devices engaged in the tube and/or of the tube in the patient body.

The tube sliding parts may also have either a constant or variable thickness and equal to or different from that of the tube.

The tube may also have a variable thickness in its longitudinal direction, and may be made by extruding or molding processes.

The membrane may be applied either to the overall profile of the tube or only to the sliding portion thereof, or also to the overall tube or only to a tube portion.

It has been found that the invention fully achieves the intended aim and objects.

In fact, the invention has provided a variable diameter tubular structure as claimed in claim 1, greatly improving surgical or implant procedures and greatly reducing the number of the introducer devices to perform the surgical procedure and the operating and bleeding times and the vessel or tissue traumas.

The structure according to the present invention allows moreover to greatly reduce the number of operations to implant artificial different diameter channels, while facilitating a closure of the puncture place with a consequent reduction of the vessel wall trauma.

## Claims

1. A biomedical variable diameter tubular structure (1), comprising an extruded and thermoformed-contour rigid tube defining a lumen of said tubular structure and made of a plastics material, said tube having a longitudinal cut defining a longitudinal region (3) extending through the overall length of said rigid tube and having overlapping radially expanding sliding walls and a resilient membrane (2) coating either the outside or the inside of said tube and extending either through said overall length of said tube or through a portion of said tube; said structure being **characterized in that** said tube **is extruded with an even thickness and** has two overlapping open end portions (4, 5) having a non linear profile comprising either inward or outward directed rough portions (6).

2. A vascular introducer device, **characterized in that** said device comprises a tubular structure (1) according to claim 1; said tubular structure further having a proximal part sealed to a valve (8) adapted to hold said tubular structure in a maximum diameter condition thereof, and an atraumatic tapering tip (9).

3. An introducer device, according to claim **2**, **characterized in that** said tubular structure has an end portion thereof coupled to a retaining valve with a side arm and an atraumatic blunted tip, the valve being a haemostatic valve comprising a stiff fitting including a silicone diaphragm insulating the outside and inside of a body vessel while allowing a medical device to pass therethrough, said silicone diaphragm being precut in the form of a star by cuts snugly adhering to said medical device.

4. An introducer device, according to claim **3**, **characterized in that** said device comprises, near said valve, a diameter transition conical region.

5. An introducer device, according to claim **2**, **characterized in that** said tubular structure is clamped by engaging therewithin a second tubular structure.

6. An introducer device, according to claim **3**, **characterized in that** said introducer device has a truncated tip, said medical device including a dilatator element having a removable tip portion adapted to protect body tissues against said introducer device truncated tip.

7. A method for making biomedical variable diameter tubular structure according to claim 1, comprising the step of:
a) providing a plastics material selected from a group of polyamide, PTFE or PE;
b) extruding said plastics material to provide a tube having a set thickness and a set rest diameter;
c) longitudinally cutting said tube thereby providing two tube open positions or edges;
d) overlapping said two tube open portions to a tube rest diameter and contour;
e) thermoforming said contour; and
f) coating either the outer or inner surface of said tube contour by a resilient membrane;
said method being **characterized in that** said tube is extruded **with an even thickness**, longitudinally cut and thermoformed providing two overlapping open end portions (4, 5) having a non linear profile comprising either inward or outward directed rough portions (6).

8. A method according to claim **7**, **characterized in that** said membrane is coated on either said outer or inner surface, with said tube in said rest diameter condition thereof, by a dipping coating step.

9. A method according to claim **7**, **characterized in that** said membrane is extruded to either said outer or inner surface.

10. A method according to claim **7**, **characterized in that** said membrane is glued either to said outer or inner surface.

## Patentansprüche

1. Biomedizinische schlauchförmige Struktur (1) mit variablem Durchmesser, die einen starren Schlauch mit einer extrudierten oder durch Thermoformen geformten Kontur umfasst, welcher ein Lumen der schlauchförmigen Struktur definiert und aus einem Kunststoffmaterial hergestellt ist, wobei der Schlauch einen Längsschnitt aufweist, der einen länglichen Bereich (3) definiert, welcher sich über die gesamte Länge von dem starren Schlauch erstreckt und überlappende, sich radial ausdehnende, ineinanderschiebbare Wände und eine elastische Dichtungsfolie (2) aufweist, welche entweder die Außenseite oder die Innenseite von dem Schlauch einhüllt und sich entweder über die gesamte Länge von dem Schlauch oder über einen Abschnitt von dem Schlauch erstreckt; wobei die Struktur **dadurch gekennzeichnet ist, dass** der Schlauch mit einer gleichmäßigen Dicke extrudiert ist und zwei überlappende offene Endabschnitte (4, 5) mit einem nicht-linearen Verlauf aufweist, die entweder nach innen oder nach außen gerichtete grobe Abschnitte (6) aufweisen.

2. Vaskuläre Einführungsvorrichtung, **dadurch gekennzeichnet, dass** die Vorrichtung eine schlauchförmige Struktur (1) nach Anspruch 1 umfasst, wobei die schlauchförmige Struktur ferner einen proximalen Teilbereich, der mit einem Ventil (8) verschlossen ist, welches eingerichtet ist, die schlauchförmige Struktur in einem Zustand mit maximalem Durchmesser davon zu halten, und eine atraumatische konisch zulaufende Spitze (9) aufweist.

3. Einführungsvorrichtung nach Anspruch 2, **dadurch gekennzeichnet, dass** ein Endabschnitt von der schlauchförmigen Struktur mit einem Halteventil mit einem Auslegerarm und einer atraumatischen stumpfen Spitze verbunden ist, wobei das Ventil ein hämostatisches Ventil ist, das einen steifen Aufsetzabschnitt umfasst, welcher eine Silikonmembran enthält, welche die Außenseite und die Innenseite eines Körpergefäßes isoliert, während sie es einer medizinischen Vorrichtung gestattet, dort hindurchzugehen, wobei die Silikonmembran, die in der Form eines Sterns durch Schnitte vorgeschnitten ist, sich passgenau an die medizinische Vorrichtung anschmiegt.

4. Einführungsvorrichtung nach Anspruch 3, **dadurch gekennzeichnet, dass** die Vorrichtung in der Nähe des Ventils einen Übergangsbereich mit einem konischen Durchmesser aufweist.

5. Einführungsvorrichtung nach Anspruch 2, **dadurch gekennzeichnet, dass** die schlauchförmige Struktur durch den Eingriff mit einer zweiten schlauchförmigen Struktur darin festgeklemmt ist.

6. Einführungsvorrichtung nach Anspruch 3, **dadurch gekennzeichnet, dass** die Einführungsvorrichtung eine gekürzte Spitze aufweist, wobei die medizinische Vorrichtung ein Dilatationselement enthält, das einen entfernbaren Spitzenbereich aufweist, der eingerichtet ist, das Körpergewebe gegen die gekürzte Spitze der Einführungsvorrichtung zu schützen.

7. Verfahren zur Herstellung einer biomedizinischen schlauchförmigen Struktur mit variablem Durchmesser nach Anspruch 1, welches die Schritte umfasst von:
a) Vorsehen eines Kunststoffmaterials, das ausgewählt ist aus der Gruppe von Polyamid, PTFE oder PE;
b) Extrudieren des Kunststoffmaterials zur Bereitstellung eines Schlauchs mit einer vorgegebenen Dicke und einem vorgegebenen Ruhedurchmesser;
c) Schneiden des Schlauchs in Längsrichtung, wodurch zwei offene Schlauchpositionen oder Schlauchränder bereitgestellt werden;
d) Überlappen der beiden offenen Schlauchabschnitte zu einem Schlauch-Ruhedurchmesser und einer Schlauchkontur;
e) Thermoformen der Kontur; und
f) Beschichten von entweder der Außen- oder der Innenfläche der Schlauchkontur mit einer elastischen Dichtungsfolie;
wobei das Verfahren **dadurch gekennzeichnet ist, dass** der Schlauch mit einer gleichmäßigen Dicke extrudiert, in Längsrichtung geschnitten und durch Thermoformen geformt ist, wodurch zwei überlappende offene Endbereiche (4, 5) bereitgestellt werden, die ein nichtlineares Profil aufweisen, welches entweder nach innen oder nach außen gerichtete grobe Abschnitte (6) umfasst.

8. Verfahren nach Anspruch 7, **dadurch gekennzeichnet, dass** die Dichtungsfolie entweder auf der Innenfläche oder auf der Außenfläche durch einen Eintauch-Beschichtungsschritt beschichtet ist, wobei der Schlauch sich in dem Zustand mit dem Ruhedurchmesser davon befindet.

9. Verfahren nach Anspruch 7, **dadurch gekennzeichnet, dass** die Dichtungsfolie entweder auf die Außen- oder auf die Innenfläche extrudiert ist.

10. Verfahren nach Anspruch 7, **dadurch gekennzeichnet, dass** die Dichtungsfolie entweder auf die Außen- oder auf die Innenfläche geklebt ist.

## Revendications

1. Structure tubulaire biomédicale à diamètre variable (1), comprenant un tube rigide à contour extrudé et thermoformé définissant une lumière de ladite structure tubulaire en matière plastique, ledit tube ayant une découpe longitudinale définissant une région longitudinale (3) s'étendant à travers la longueur totale dudit tube rigide et ayant des parois coulissantes s'étendant radialement et se chevauchant, et une membrane résiliente (2) revêtant l'extérieur ou l'intérieur dudit tube et s'étendant à travers ladite longueur totale dudit tube ou à travers une partie dudit tube ; ladite structure étant **caractérisée en ce que** ledit tube **est extrudé avec une épaisseur égale** et comporte deux parties d'extrémité ouvertes et se chevauchant (4, 5) ayant un profil non linéaire comprenant des parties rugueuses orientées vers l'intérieur ou l'extérieur (6).

2. Dispositif introducteur vasculaire, **caractérisé en ce que** ledit dispositif comprend une structure tubulaire (1) selon la revendication 1, ladite structure tubulaire ayant en outre une partie proximale scellée à une valve (8) conçue pour maintenir ladite structure tubulaire dans une condition de diamètre maximum de celle-ci, et une extrémité conique atraumatique (9).

3. Dispositif introducteur, selon la revendication **2**, **caractérisé en ce que** ladite structure tubulaire comporte une partie d'extrémité de celle-ci couplée à une valve de retenue avec un bras latéral et une extrémité émoussée atraumatique, la valve étant une valve hémostatique comprenant un raccord rigide incluant un diaphragme en silicone isolant l'extérieur et l'intérieur d'un vaisseau du corps tout en permettant le passage d'un dispositif médical à travers, ledit diaphragme en silicone étant prédécoupé sous forme d'une étoile, les découpes adhérant aisément audit dispositif médical.

4. Dispositif introducteur, selon la revendication **3, caractérisé en ce que** ledit dispositif comprend, près de ladite valve, une région conique de transition de diamètre.

5. Dispositif introducteur, selon la revendication **2, caractérisé en ce que** ladite structure tubulaire est immobilisée en s'engageant à l'intérieur d'une seconde structure tubulaire.

6. Dispositif introducteur, selon la revendication **3, caractérisé en ce que** ledit dispositif introducteur présente une extrémité tronquée, ledit dispositif médical incluant un élément dilatateur ayant une partie d'extrémité amovible conçue pour protéger les tissus corporels de l'extrémité tronquée dudit dispositif introducteur.

7. Procédé de réalisation d'une structure tubulaire biomédicale à diamètre variable selon la revendication 1, comprenant les étapes de :
a) fourniture d'une matière plastique choisie dans un groupe de polyamide, PTFE ou PE ;
b) extrusion de ladite matière plastique pour former un tube ayant une épaisseur définie et un diamètre au repos défini.
c) découpe longitudinale dudit tube, fournissant de ce fait deux positions d'ouverture du tube ou bords ;
d) chevauchement desdites parties ouvertes du tube sur un diamètre au repos du tube et un contour ;
e) thermoformage dudit contour ; et
f) revêtement de la surface extérieure ou intérieure dudit contour de tube par une membrane résiliente ;
ledit procédé étant **caractérisé en ce que** ledit tube est extrudé **avec une épaisseur égale,** découpé longitudinalement et thermoformé pour fournir deux parties d'extrémité ouvertes se chevauchant (4, 5), et ayant un profil non linéaire comprenant des parties rugueuses orientées vers l'intérieur ou l'extérieur (6).

8. Procédé selon la revendication **7, caractérisé en ce que** ladite membrane est revêtue sur ladite surface intérieure ou extérieure, ledit tube étant dans ladite condition de diamètre au repos de celui-ci, en procédant à une étape de revêtement par immersion.

9. Procédé selon la revendication **7, caractérisé en ce que** ladite membrane est extrudée sur ladite surface intérieure ou extérieure.

10. Procédé selon la revendication **7, caractérisé en ce que** ladite membrane est collée sur ladite surface intérieure ou extérieure.
